# EUROPEAN PATENT APPLICATION

(11) **EP 3 998 015 A1**
(43) Date of publication of application: **18.05.2022**
(21) Application number: 19937090.9
(22) Date of filing: 10.07.2019
(51) Int. Cl.: A61B 1/045

(54) **IMAGE ANALYZING DEVICE AND IMAGE ANALYZING METHOD**

(71) Applicant: Cybernet Systems Co., Ltd., Tokyo 101-0022 (JP); Showa University, Tokyo 142-8555 (JP)
(72) Inventor: MISAWA, Masashi, Yokohama, Kanagawa 224-8503 (JP); MORI, Yuichi, Yokohama, Kanagawa 224-8503 (JP); KUDO, Shin-ei, Yokohama, Kanagawa 224-8503 (JP); WAKISAKA, Takashi, Tokyo 101-0022 (JP); KAHARA, Hideo, Tokyo 101-0022 (JP)
(74) Representative: v. Bezold & Partner Patentanwälte - PartG mbB
(86) International application number: PCT/JP2019/027273
(87) International publication number: WO 2021/005733

(57) **Abstract**

This disclosure has an object to allow an automatic discrimination between a super enlarged image and a non-enlarged image in a computer diagnosis assistance system that analyzes a state of an epithelium using an image analysis. An image analyzing device (10) according to this disclosure is the image analyzing device (10) to be connected to an endoscope (20), and includes a target image determination unit (111) that obtains an image from the endoscope (20) and determines whether or not the image is a target image using a halation region included in the image, and an image analyzing unit (112) that analyzes a state of an epithelium, captured by the endoscope (20), using the target image when the image is the target image.

## Description

### TECHNICAL FIELD

This disclosure relates to an image analyzing device and an image analyzing method.

### BACKGROUND ART

Recently, an endoscope with a super-magnifying function having a microscope level magnification of 380 times or more has been developed, and an endoscope Endocytoscopy that can observe an epithelium of a body lumen in a living body by magnifying it to a level of a cell nucleus and a cell of a blood vessel, a glandular cavity, and the like. Endocytoscopy is a kind of a contact-type endoscope, and brings a lens surface into contact with an epithelium as a target and uses a zoom mechanism mounted to the endoscope to adjust the focus, thereby obtaining a super enlarged image. For the super enlarged image, the usability in the prediction of the histopathological diagnosis of an organ, such as a gullet (for example, see Non-Patent Literature 1), a stomach (for example, see Non-Patent Literature 2), a duodenum (for example, see Non-Patent Literature 3), and a large bowel (for example, see Non-Patent Literature 4) has been reported.

However, even when the super enlarged image is captured using Endocytoscopy, the proficiency at a certain level or more in the image analysis of the super enlarged image is necessary for performing the prediction of the histopathological diagnosis (for example, see Non-Patent Literature 4), and then a computer diagnosis assistance system has been developed to allow the prediction of the histopathological diagnosis without the proficiency at a certain level or more. It has been proved that this is effective for the prediction of the histopathological diagnosis (for example, see Non-Patent Literatures 5 and 6).

### CITATION LIST

### Non-Patent Literature

Non-Patent Literature 1: Y. Kumagai, K. Monma, K. Kawada, "Magnifying chromoendoscopy of the esophagus: in-vivo pathological diagnosis using an endocytoscopy system", Endoscopy 2004; 36:590-4.
Non-Patent Literature 2: H. Sato, H. Inoue, B. Hayee, et al., "In vivo histopathology using endocytoscopy for non-neoplastic changes in the gastric mucosa: a prospective pilot study (with video)", Gastrointest Endosc 2015; 81:875-81.
Non-Patent Literature 3: S. Miyamoto, T. Kudo, S. Abiko, et al., "Endocytoscopy of Superficial Nonampullary Duodenal Epithelial Tumor: Two Cases of Tubular Adenocarcinoma and Adenoma", Am J Gastroenterol 2017; 112:1638.
Non-Patent Literature 4: SE Kudo, K. Wakamura, N. Ikehara, et al., "Diagnosis of colorectal lesions with a novel endocytoscopic classification - a pilot study", Endoscopy 2011; 43:869-75.
Non-Patent Literature 5: Y. Mori, S. Kudo, K. Wakamura, et al., "Novel computer-aided diagnostic system for colorectal lesions by using endocytoscopy (with videos)", Gastrointestinal Endoscopy 2015; 81:621-629.
Non-Patent Literature 6: M. Misawa, S. Kudo, Y Mori, et al., "Characterization of colorectal lesions using a computer-aided diagnostic system for narrow-band imaging endocytoscopy", Gastroenterology 2016; 150:1531-1532.

### SUMMARY OF INVENTION

### Technical Problem

Endocytoscope can also take a non-enlarged image smaller than the super enlarged image in magnification. Therefore, for applying the computer diagnosis assistance system to Endocytoscopy, it is necessary to discriminate between the super enlarged images and the non-enlarged images among the images captured by the endoscope. However, a technique to automatically discriminate between the super enlarged image and the non-enlarged image is not present. Therefore, an operator of the system has been required to determine a super enlarged image as a target to be subjected to an image analysis of a state of an epithelium from the images captured by Endocytoscopy and input it to the system.

While it is considered that a switch or a button exclusive for the input to the system is disposed, adding such a switch or a button is not preferred. Meanwhile, when automatization of the determination of the super enlarged image by the operator of the system becomes available, the operation of the system is more facilitated, thus leading to reduction of the burden on a patient. Therefore, this disclosure has an object to allow an automatic discrimination between a super enlarged image and a non-enlarged image in a computer diagnosis assistance system that analyzes a state of an epithelium using an image analysis.

### Solution to Problem

Since the super enlarged image is an image of a contact-type endoscope, a halation of a light source does not occur in the image. This disclosure is focused on the halation of the light source, and determines the image as a super enlarged image when the halation is not detected in the image. Accordingly, this disclosure allows the automatic discrimination between the super enlarged image and the non-enlarged image, and allows automatically selecting a target image to be subjected to the image analysis in the computer diagnosis assistance.

An image analyzing device according to this disclosure is an image analyzing device to be connected to an endoscope, and the image analyzing device includes a target image determination unit that obtains an image from the endoscope and determines whether or not the image is a target image using a halation region included in the image, and an image analyzing unit that analyzes a state of an epithelium, captured by the endoscope, using the target image when the image is the target image.

An image analyzing method according to this disclosure is an image analyzing method executed by an image analyzing device connected to an endoscope, and the image analyzing device executes a target image determining step of obtaining an image from the endoscope and determining whether or not the image is a target image using a halation region included in the image, and an image analyzing step of analyzing a state of an epithelium, captured by the endoscope, using the target image when the image is the target image.

An image analyzing program according to this disclosure is a program that causes a computer to achieve each of the functional units included in the image analyzing device according to this disclosure and is a program that causes a computer to execute each of the steps included in the image analyzing method according to this disclosure, and the program may be recorded in a computer readable recording medium.

### Advantageous Effects of Invention

According to this disclosure, since a super enlarged image and a non-enlarged image can be automatically discriminated in a computer diagnosis assistance system that analyzes a state of an epithelium using the super enlarged image, an image to be an analysis target of an image analysis can be automatically selected. That is, a region of interest (Region of Interest: ROI) to be an analysis target that requires a setting in the computer diagnosis assistance system can be automatically selected. Therefore, the operation of the system can be more facilitated, and a burden on a patient can be reduced. Additionally, since this disclosure automatically selects the target image of the computer diagnosis assistance, a period necessary for outputting the prediction result of the histopathological diagnosis can be shortened.

### BRIEF DESCRIPTION OF DRAWINGS

Fig. 1 illustrates an example of a computer diagnosis assistance system according to the embodiment.
Fig. 2 illustrates an example of a configuration of a distal end portion of an endoscope.
Fig. 3 illustrates a first example of an image captured by an imaging device.
Fig. 4 illustrates a second example of the image captured by the imaging device.
Fig. 5 illustrates a third example of the image captured by the imaging device.
Fig. 6 illustrates a schematic diagram of a cell nucleus.

### DESCRIPTION OF EMBODIMENTS

The following describes embodiments of this disclosure in detail with reference to the drawings. Note that this disclosure is not limited to the embodiments described below. These embodiments are merely examples, and this disclosure can be executed in configurations in which various kinds of changes and improvements are performed based on knowledge of those skilled in the art. Note that components with the same reference numeral in the specification and the drawings should mutually indicate the same component.

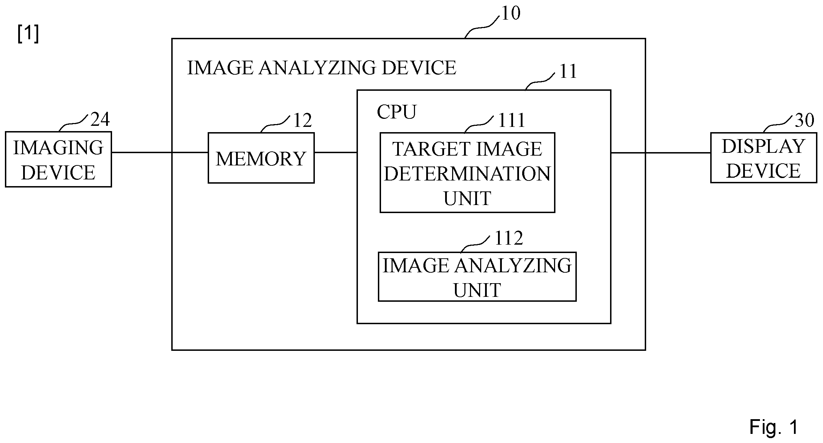

Fig. 1 illustrates an example of a computer diagnosis assistance system according to the embodiment. The computer diagnosis assistance system according to the embodiment includes an image analyzing device 10, an imaging device 24, and a display device 30. The image analyzing device 10 includes a CPU (Central Processing Unit) 11 and a memory 12. The CPU 11 functions as a target image determination unit 111 and an image analyzing unit 112. The display device 30 may be included in the image analyzing device 10.

The image analyzing device 10 may be achieved by executing a computer program stored in the memory 12. The computer program is a program to cause a computer to execute each of steps included in an image analyzing method according to this disclosure. In the image analyzing method according to this disclosure, the image analyzing device 10 executes a target image determining step and an image analyzing step.

In the target image determining step, the target image determination unit 111 obtains an image from an endoscope, and uses a halation region included in the image to determine whether or not the image is a target image. When the image is the target image, the image analyzing unit 112 executes the image analyzing step. In the image analyzing step, the image analyzing unit 112 uses the target image to analyze a state of an epithelium captured by the endoscope.

The imaging device 24 is any imaging device mounted to the endoscope, and for example, a CCD (Charge Coupled Device) can be exemplified. The imaging device 24 has a function of capturing a moving image, and also has a function of capturing a still image. Therefore, the image captured by the imaging device 24 includes not only the moving image but also the still image. When the image captured by the imaging device 24 is obtained, the CPU 11 displays the image on the display device 30.

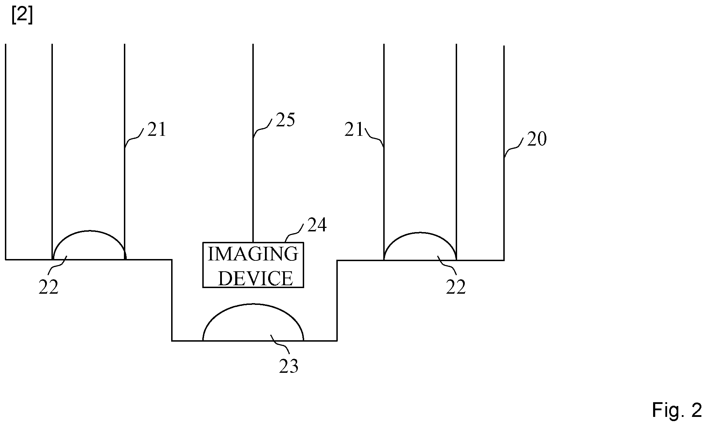

Fig. 2 illustrates an example of a configuration of a distal end portion of an endoscope. At a distal end of an endoscope 20, light guide lenses 22 and an objective lens 23 are disposed. The objective lens 23 is disposed at a projecting portion in the distal end of the endoscope 20, and the light guide lenses 22 are disposed at positions lower than that of the objective lens 23.

An irradiating light output from a light source device (not illustrated) is emitted from the light guide lenses 22 via light guides 21. An image of an epithelium of a body lumen irradiated with the irradiating light is guided to the imaging device 24 passing through the objective lens 23. Thus, the image of the epithelium of the body lumen is captured by the imaging device 24.

The image captured by the imaging device 24 is transmitted to the image analyzing device 10 using a signal line 25. In this transmitting, the image may be transmitted to the image analyzing device 10 using a wireless communication function unit (not illustrated) mounted to the imaging device 24. Between the objective lens 23 and the imaging device 24, one or more lenses may be disposed while they are omitted in Fig. 2. While Fig. 2 illustrates an example in which the distal end portion of the endoscope 20 is provided with the projecting portion, this disclosure is not limited thereto, and for example, the distal end portion of the endoscope 20 may be flat and the objective lens 23 and the light guide lenses 22 may be disposed on the flat surface.

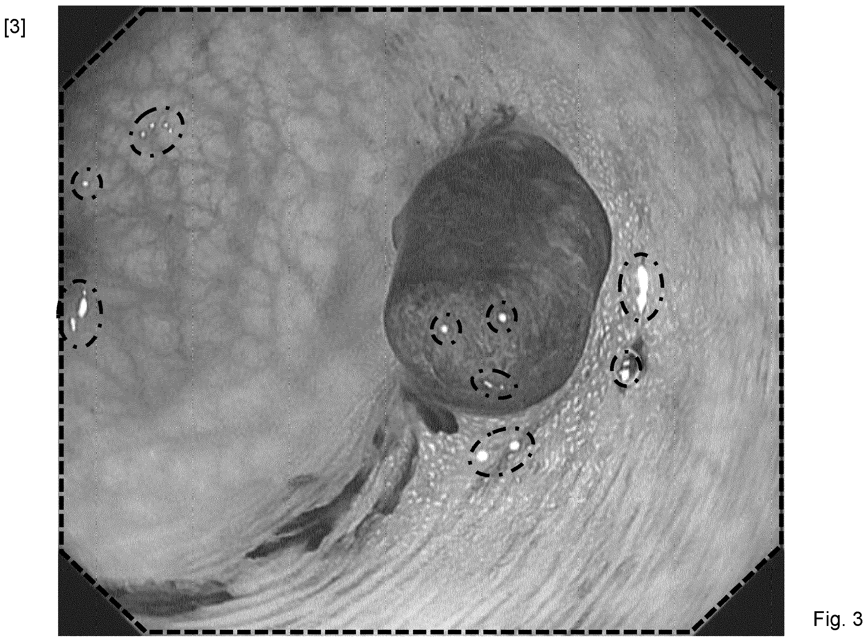

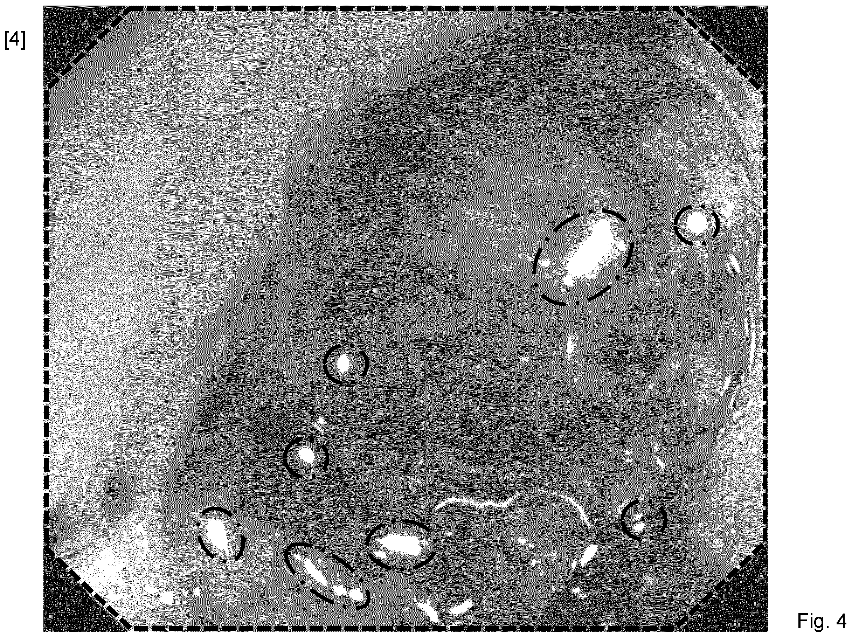

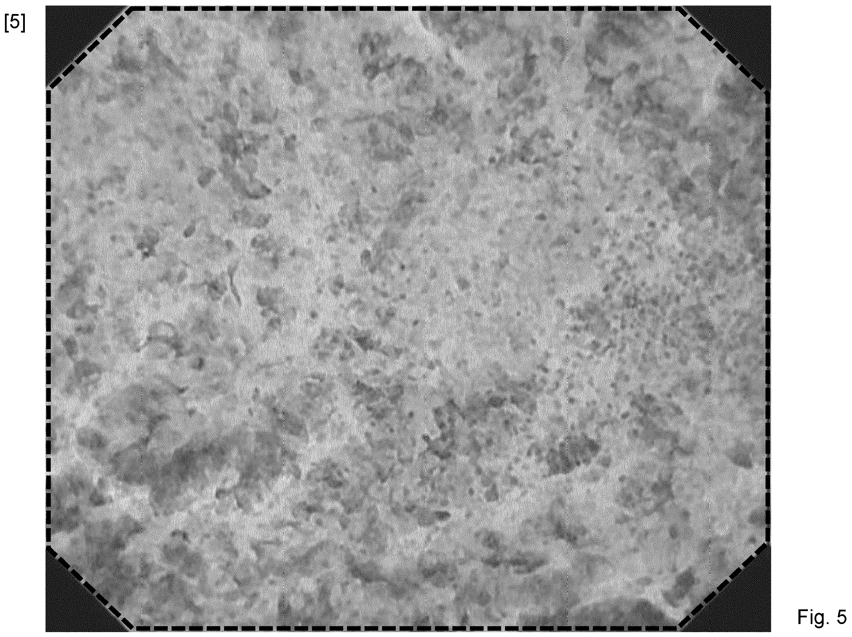

Fig. 3, Fig. 4, and Fig. 5 illustrate examples of an image captured by the imaging device 24. The image illustrated in Fig. 4 indicates an image of a part of the image of Fig. 3 with the part enlarged and focused. The image illustrated in Fig. 5 indicates a super enlarged image of a part of the image of Fig. 4 with the part further enlarged and focused. For applying the computer diagnosis assistance system to the prediction of the histopathological diagnosis, an observation of a pathological tissue super enlarged to a cell level is indispensable. Therefore, while it is necessary to discriminate between super enlarged images and non-enlarged images among the images captured by the endoscope 20, the endoscope 20 can ordinarily capture not only the super enlarged image but also the non-enlarged image of a normal magnification as illustrated in Fig. 3 and Fig. 4.

When an operator of the computer diagnosis assistance system finds a site suspected to be a lesion in a video displayed in the display device 30, the operator captures sequentially enlarged still images as illustrated in Fig. 3, Fig. 4, and Fig. 5. In the images illustrated in Fig. 3 and Fig. 4, since an ROI and other portions are included, it is necessary to set the ROI to perform the image analysis. Meanwhile, the super enlarged image illustrated in Fig. 5 is an image of taking the ROI itself without the regions other than the ROI.

While the non-enlarged image needs for the operator of the system to set the ROI, the super enlarged image does not need the setting of the ROI because it is an image in which the ROI itself is captured. Therefore, by automatically determining the super enlarged image, the ROI image to be subjected to the image analysis can be automatically selected.

When the imaging device 24 captures an image in a state where the objective lens 23 illustrated in Fig. 2 is out of contact with an epithelium, an image of the light guide lenses 22 is reflected on the surface of the epithelium to be projected in the imaging device 24. Therefore, in the image in the state where the objective lens 23 is out of contact with the mucosal epithelium, as indicated by regions surrounded by one dot chain lines in Fig. 3 and Fig. 4, regions in which halation occurs are present.

Meanwhile, when the super enlarged image is captured, since the objective lens 23 illustrated in Fig. 2 is in contact with the epithelium, the image of the light guide lenses 22 reflected by the surface of the epithelium is not projected in the imaging device 24. Any lights entering the imaging device 24 are lights that have passed through cells of the epithelium. Therefore, in the super enlarged image illustrated in Fig. 5, the regions in which the halation occurs as illustrated in Fig. 3 and Fig. 4 are not generated, and the number of pixels in the halation region becomes a certain ratio or less. Here, the certain ratio is, for example, 0.0000077% or less.

Therefore, the target image determination unit 111 obtains an image from the imaging device 24, and determines whether the image is a super enlarged image captured from the transmitted light transmitted through the cells of the epithelium or not using the halation region included in the image. For example, since the halation regions are present in the images illustrated in Fig. 3 and Fig. 4, the target image determination unit 111 determines them not to be the target images. Meanwhile, since the halation region is not present in the image illustrated in Fig. 5, the target image determination unit 111 determines it to be the target image. Accordingly, this disclosure allows automatically predicting the histopathological diagnosis of the ROI by selecting the super enlarged image and performing the image analysis of the image.

Here, to the image analyzing device 10, a video and a still image from the endoscope 20 are input. In this disclosure, the image to be subjected to the image analysis is the super enlarged image. Therefore, the target image determination unit 111 preferably determines whether the image obtained from the endoscope 20 is a still image or not, and preferably determines whether the image is the target image or not when the image is the still image.

When the image obtained from the endoscope 20 is the super enlarged image, the image is an image in which the ROI is captured. Therefore, when the image is the target image, the image analyzing unit 112 stores the image determined to be the target image in the memory 12 as an image in which the ROI is captured. Accordingly, the system according to this disclosure allows efficiently collecting information on the ROI.

When the image is the target image, the image analyzing unit 112 performs the image analysis using the target image to analyze the state of the epithelium captured by the imaging device 24. The image analyzing unit 112 predicts the histopathological diagnosis using an analysis result of the state of the epithelium. The prediction of the histopathological diagnosis is, for example, discrimination among a non-tumor, an adenoma, and a cancer. The prediction of the histopathological diagnosis may include a sessile serrated adenoma/polyp (SSA/P) that possibly becomes a tumor. The CPU 11 outputs the analysis result of the image analyzing unit 112 to the display device 30, and the display device 30 displays a prediction result of the histopathological diagnosis. The CPU 11 further stores the analysis result of the image analyzing unit 112 in the memory 12.

A machine learning is preferably used for the prediction of the histopathological diagnosis, and accordingly, the histopathological diagnosis prediction without the need for professional training can be achieved using the computer diagnosis assistance system. In this case, for the histopathological diagnosis prediction, data as a learning sample is provided to the image analyzing device 10 for each of the non-tumor, the adenoma, the cancer, and the SSA/P

As the machine learning, for example, SVM (Support Vector Machine), a neural network, Naive Bayes Classifier, a decision tree, a cluster analysis, a linear regression analysis, a logistic regression analysis, and a random forest are usable. The neural network may be a structured learning (deep learning) using a multi-layered neural network.

The image analyzing unit 112 may use a non-enlarged image in the image analysis. For example, when analyzing the super enlarged image illustrated in Fig. 5, at least any image of Fig. 3 and Fig. 4 is used. The non-enlarged image includes the regions other than the ROI. Therefore, the image analyzing unit 112 obtains a region setting of the ROI in the non-enlarged image input to the image analyzing device 10, and uses the image of the region determined by the region setting for the image analysis.

The following describes a specific example of the determination by the target image determination unit 111 whether the halation region is present or not.

In the determination whether the halation region is present or not, the image captured by the imaging device 24 is extracted, and the number of pixels where the halation occurs included in the extracted pixels is counted. Then, a determination of the super enlarged image, that is, an image of the analysis target is made when the number of pixels where the halation occurs in the extracted pixels is a preliminarily determined certain ratio or less, and a determination of a non-enlarged image is made when the number of pixels where the halation occurs in the extracted pixels exceeds the preliminarily determined certain ratio.

Here, the extraction of the image captured by the imaging device 24 means, for example, to extract regions surrounded by dashed lines illustrated in Fig. 3 to Fig. 5. While the certain ratio is any ratio, for example, 0.0000077% or less described above is usable.

The determination of whether the halation region or not is made based on, for example, whether a luminance exceeds a predetermined value or not. For example, when color information for each color (R value, G value, B value) of each pixel has a gradation of 255 levels, the halation region is determined when the colors each become 240 or more. This determination only needs to extract a white region, and is not limited thereto. For example, this determination may be performed with the luminance of white light obtained by combination of the color information (R value, G value, B value), and may be performed with a color space indicated by a color phase, a chroma, and a brightness.

In the epithelium observation using an endoscope, a wavelength of the light emitted from the light guide lens 22 and a wavelength of the light to be captured by the imaging device 24 differ in some cases. For example, a case where the epithelium observation with white light is performed and a case where a narrowband light observation (NBI: Narrow Band Imaging, BLI: Blue Laser Imaging) is performed are included in the cases. Also for a light source of the light emitted from the light guide lens 22, various kinds of light sources, such as a xenon light source, a laser light source, a halogen light source, and a LED (Light Emitting Diode), are used. Therefore, the threshold for determining the halation region is preferably set depending on the wavelength of the irradiating light emitted from the light guide lens 22 and the wavelength captured by the imaging device 24.

For example, when the irradiating light emitted from the light guide lens 22 is a white light, the target image determination unit 111 makes a determination of the halation region when the color information for each color (R value, G value, B value) becomes 240 or more. For example, in the case of the narrowband light observation (NBI: Narrow Band Imaging, BLI: Blue Laser Imaging), the target image determination unit 111 determines the halation region when the color information for color (R value, G value, B value) becomes 200 or more, 240 or more, and 180 or more, respectively.

The following describes the image analysis using the target image in the image analyzing unit 112 in detail.

The image analysis using the target image can include, for example, a texture analysis. In the texture analysis, an image of an epithelium as indicated by a dashed line in Fig. 5 is extracted, and the analysis is performed on the extracted image. While the method for the texture analysis is any method, it is preferably one configured to analyze a local image feature quantity usable for detecting an object and a face. As such an analysis method, for example, a SIFT (Scale-Invariant Feature Transform), a SURF (Speed-Upped Robust Feature), and a Haar-Like feature can be adopted.

The image analysis using the target image can include, for example, an analysis of the feature quantity obtained from the super enlarged image. The feature quantity obtained from the image is, for example, feature quantities of a cell nucleus, a blood vessel, and a glandular cavity.

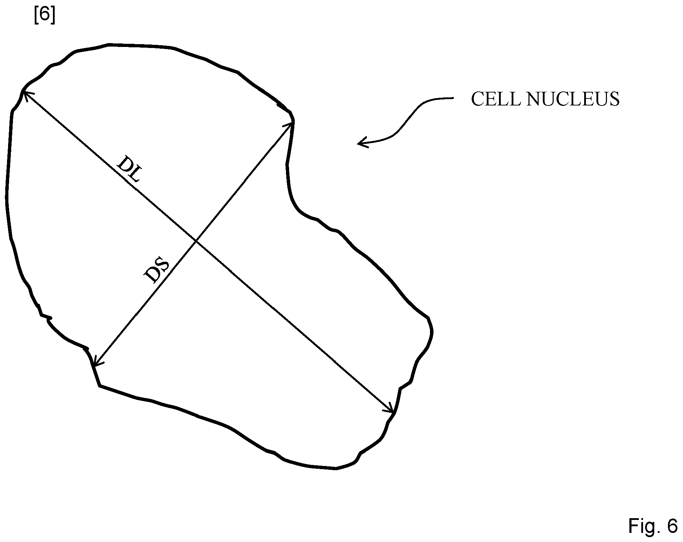

Fig. 6 illustrates a schematic diagram of a cell nucleus. The feature quantity of the cell nucleus can include, for example, a major axis DL of the cell nucleus, a minor axis DS of the cell nucleus, a perimeter of the cell nucleus, an area of the cell nucleus, a roundness of the cell nucleus, and a color of the cell nucleus. The feature of the cell nucleus may include an eccentricity, a pitch-chord ratio, an uneven shape, a fractal dimension, a line concentration, and a density contrast.

When the feature quantity of the cell nucleus is used, the image analyzing unit 112 extracts a cell nucleus included in the image. The method for extracting the cell nucleus is any method that performs it by, for example, a segmentation of a region of the cell nucleus and an artifact removal. For the segmentation of the region of the cell nucleus, for example, Otsu's binarization method with the R component is used. In the artifact removal, for example, pixels in which white pixels are continuous in a binarized image are defined as one region and the area, the major axis, and the roundness are calculated for each region. A region where the area is in a set range (for example, from 30 µm² to 500 µm²), the major axis is a set value (for example, 30 µm or less), and the roundness is a set value (for example, 0.3 or more) is left as the analysis targets, and the other regions are removed. The major axis and the roundness are calculated by, for example, an elliptical approximation of the region. When the number of the extracted nuclei is a preliminarily set number (for example, 30) or less, the extracted nuclei may be removed from the feature quantity of the analysis target.

While the feature quantity of the cell nucleus may be the feature quantity of a part of cell nuclei included in the target image, the features of all the cell nuclei are preferably measured. The feature quantity of the cell nucleus preferably includes an average value and a standard deviation calculated from the features of the cell nuclei included in the target image.

The feature quantity of the blood vessel is, for example, the largest diameter of a largest blood vessel, a ratio between the smallest and the largest diameters of the largest blood vessel, and a proportion of blood vessel region occupied in the whole image. When the feature quantity of the blood vessel is used, the image analyzing unit 112 extracts blood vessel regions included in the image. The method for extracting the blood vessel region is any method. Extracting the blood vessel region can be executed by, for example, making linearity images, synthesizing a plurality of the linearity images to make a blood vessel candidate region image, and removing a region that is not the blood vessel region from the image.

The feature quantities of the cell nucleus and the blood vessel are applicable also to the image analysis targeted to any organ such as an oral cavity, a pharynx, a larynx, a gullet, a stomach, a duodenum, a jejunum, an ileum, a large bowel, a trachea, a bile duct, a pancreas duct, a uterus, a bladder, and a urinary duct.

For the stomach and the large bowel, a glandular cavity can be observed in the super enlarged image. Therefore, in the prediction of the histopathological diagnosis of the stomach and the large bowel, the image analyzing unit 112 preferably analyzes the feature quantity of the glandular cavity. The feature quantity of the glandular cavity can include, for example, a major axis of the glandular cavity, a minor axis of the glandular cavity, a perimeter of the glandular cavity, an area of the glandular cavity, a roundness of the glandular cavity, and a color of the glandular cavity.

For the duodenum, the jejunum, and the ileum, a villous structure can be observed in the super enlarged image. Therefore, in the prediction of the histopathological diagnosis of the duodenum, the jejunum, and the ileum, the image analyzing unit 112 preferably analyzes the feature quantity of the villous structure. The feature quantity of the villous structure can include, for example, a major axis of a villus tip, a minor axis of the villus tip, and the number of villi per visual field.

Thus, the image analyzing unit 112 preferably analyzes the feature quantity of the glandular cavity or the villous structure in addition to the cell nucleus and the blood vessel in a columnar epithelium region, and preferably analyzes the feature quantities of the nucleus and the blood vessel in a stratified squamous epithelium, a ciliated epithelium other than the columnar epithelium region.

Here, information for the image indicating which of the cell nucleus, the blood vessel, the glandular cavity, and the villous structure is focused on is not attached to the image obtained from the endoscope 20. Therefore, the image analyzing unit 112 preferably determines which of the cell nucleus, the blood vessel, the glandular cavity, and the villous structure is captured in the image before extracting the features of the cell nucleus, the blood vessel, the glandular cavity, and the villous structure. For example, the image analyzing unit 112 extracts each of the cell nucleus, the blood vessel, the glandular cavity, and the villous structure from the image, and extracts the feature quantity of the extracted one. Accordingly, an operation amount in the image analysis is reduced, and a period necessary for the prediction of the histopathological diagnosis can be shortened.

Here, for the organs other than the stomach and the large bowel, the glandular cavity is not observed in a normal state. However, due to the generation of a tumor, the glandular cavity appears even in the organs other than the stomach and the large bowel in some cases. Therefore, the image analyzing unit 112 preferably analyzes the feature quantity of the glandular cavity also for the organs other than the stomach and the large bowel.

For the organs other than the duodenum, the jejunum, and the ileum, the villous structure is not observed in a normal state. However, due to the generation of a tumor, the villous structure appears even in the organs other than the duodenum, the jejunum, and the ileum in some cases. Therefore, the image analyzing unit 112 preferably analyzes the feature quantity of the villous structure also for the organs other than the duodenum, the jejunum, and the ileum.

As described above, since this disclosure allows the automatic discrimination between the super enlarged image and the non-enlarged image, the ROI can be automatically discriminated and the computer diagnosis assistance system that automatically predicts the histopathological diagnosis using the super enlarged image can be provided.

### REFERENCE SIGNS LIST

- 10: Image analyzing device
- 11: CPU
- 111: Target image determination unit
- 112: Image analyzing unit
- 12: Memory
- 20: Endoscope
- 21: Light guide
- 22: Light guide lens
- 23: Objective lens
- 24: Imaging device
- 25: Signal line
- 30: Display device

## Claims

1. An image analyzing device to be connected to an endoscope, comprising:
a target image determination unit that obtains an image from the endoscope and determines whether or not the image is a target image using a halation region included in the image; and
an image analyzing unit that analyzes a state of an epithelium, captured by the endoscope, using the target image when the image is the target image.

2. The image analyzing device according to claim 1, wherein
the target image is an image captured by using a transmitted light that have passed through cells of the epithelium.

3. The image analyzing device according to claim 1 or 2, wherein
the target image is an image captured in a state where an objective lens included in the endoscope is in contact with the epithelium.

4. The image analyzing device according to any of claims 1 to 3, wherein
the target image determination unit stores the image determined to be the target image in a memory as an image in which a region of interest is captured.

5. The image analyzing device according to any of claims 1 to 4, wherein
the target image is an image in which at least any of a cell nucleus, a blood vessel, a glandular cavity, and a villous structure is captured.

6. The image analyzing device according to claim 5, wherein
the image analyzing unit includes a process of extracting a feature quantity of at least any of the cell nucleus, the blood vessel, the glandular cavity, and the villous structure from the target image, and analyzes the state of the epithelium using an extraction result.

7. The image analyzing device according to claim 5 or 6, wherein
the image analyzing unit determines which of the cell nucleus, the blood vessel, the glandular cavity, and the villous structure is captured in the image.

8. The image analyzing device according to any of claims 1 to 7, wherein
the image analyzing unit predicts a histopathological diagnosis using an analysis result of the state of the epithelium.

9. The image analyzing device according to claim 8, wherein
the prediction of the histopathological diagnosis is a discrimination among a non-tumor, an adenoma, and a cancer.

10. The image analyzing device according to any of claims 1 to 9, wherein
the target image determination unit determines that the image is the target image when a number of pixels of the halation region included in the image is a certain ratio or less.

11. The image analyzing device according to any of claims 1 to 10, wherein
the target image determination unit determines whether the image obtained from the endoscope is a still image or not, and determines whether the image is the target image or not in a case of the still image.

12. A program that causes a computer to achieve each of the functional units included in the image analyzing device according to any of claims 1 to 11.

13. An image analyzing method executed by an image analyzing device connected to an endoscope, the method comprising:
a target image determining step of obtaining an image from the endoscope and determining whether or not the image is a target image using a halation region included in the image; and
an image analyzing step of analyzing a state of an epithelium, captured by the endoscope, using the target image when the image is the target image.
